## Europäisches Patentamt
## European Patent Office
## Office européen des brevets

(19)

(11) Numéro de publication: **0 273 010 B1**

(12)

# FASCICULE DE BREVET EUROPEEN

(45) Date de publication de fascicule du brevet:
**02.10.91**

(51) Int. Cl.⁵: **A61B 5/08**, A61B 5/113,
A61D 3/00, A01K 1/06

(21) Numéro de dépôt: **87810763.0**

(22) Date de dépôt: **18.12.87**

(54) **Enceinte de mesure pléthysmographique.**

(30) Priorité: **22.12.86 CH 5128/86**

(43) Date de publication de la demande:
**29.06.88 Bulletin 88/26**

(45) Mention de la délivrance du brevet:
**02.10.91 Bulletin 91/40**

(84) Etats contractants désignés:
**AT BE CH DE ES FR GB GR IT LI LU NL SE**

(56) Documents cités:
EP-A- 0 121 795
DE-A- 2 251 216
DE-A- 2 837 005
GB-A- 944 998
US-A- 2 789 538

ISA TRANSACTIONS, vol. 15, no. 2, 1976,
pages 161-166, Pittsburgh, P.A., US; J.R. DEC-
KER et al.: "Automated rodent respiratory
monitor and histogram computer-a preliminary report"

(73) Titulaire: **RESEARCH AND CONSULTING COM-
PANY AG**

**CH-4452 Itingen(CH)**

(72) Inventeur: **Nikiforow, Andrey**
**26, Olive Court**
**Flemington, N.J. 08822(US)**
Inventeur: **Ventrone, Roger**
**Champ Cru**
**Lucinges F-74380 Bonne(FR)**

(74) Mandataire: **Dousse, Blasco et al**
**7, route de Drize**
**CH-1227 Carouge/Genève(CH)**

## Description

La présente invention se rapporte à une enceinte de mesure pléthysmographique destinée à recevoir le corps d'un animal de laboratoire en vue de mesurer différents paramètres respiratoires, cette enceinte comprenant un corps tubulaire dont l'extrémité avant présente une section tronconique se terminant par une ouverture d'admission d'un flux gazeux à inhaler, cette enceinte étant délimitée d'une part vers cette ouverture d'admission par deux joints annulaires séparés axialement, dont les ouvertures respectives sont destinées au passage du museau de l'animal, d'autre part par un piston d'adaptation du volume de l'enceinte à la taille de l'animal.

De telles enceintes servent à déterminer différents paramètres respiratoires d'un animal de laboratoire soumis à des tests d'inhalation de gaz ou d'aérosols formés de différentes substances. Le flux gazeux consécutif aux variations de volume du corps de l'animal est mesuré par un transducteur et le signal ainsi obtenu est traité en fonction des paramètres que l'on désire connaître. Ce mode de mesure et de traitement de signal capté est décrit en détail dans un article "Measurement of respiratory patterns in rodents using whole-body plethymography and a pneumotachograph" publié dans le No 15 de Laboratory Animals (1981), pages 137-140.

Il est évident que la précision de la mesure ne peut être assurée que si le volume de gaz de l'enceinte pléthysmographique ne varie qu'en fonction des variations de volume du corps de l'animal, consécutives au volume de gaz ou d'aérosol inhalé. Dans le cas où le gaz ou l'aérosol à inhaler est formé de substances irritantes, l'animal cherche instinctivement à échapper au flux de gaz ou d'aérosol qui lui est envoyé par l'ouverture d'admission du corps tubulaire vers laquelle son museau est dirigé. Ces tentatives de recul de l'animal qui sont limitées par le piston, induisent cependant des variations erratiques de volume dans l'enceinte phléthysmographique et donc des bruits importants dans le signal enregistré par le transducteur. Ces bruits sont difficiles à filtrer de sorte qu'ils faussent les paramètres calculés et par conséquent l'évaluation des conditions respiratoires de l'animal soumis au test d'inhalation.

Le but de la présente invention est de remédier, au moins en partie, à ces inconvénients afin de rendre la mesure plus précise et donc plus fiable.

A cet effet, cette invention a pour objet une enceinte de mesure pléthysmographique telle que définie par la revendication 1.

Le rôle du collier est d'empêcher l'animal de reculer sa tête et ainsi d'augmenter sensiblement le volume occupé dans l'enceinte pléthysmographique, lorsqu'il cherche à se soustraire au courant de gaz ou d'aérosol distribué à travers l'ouverture d'admission. En effet, il n'est pas possible de comprimer l'animal à l'aide du piston. Celui-ci n'est destiné qu'à limiter le volume de l'enceinte pléthysmographique à la taille de l'animal, mais pas à le comprimer, auquel cas il ne pourrait plus respirer normalement. En l'empêchant de reculer sa tête à l'aide du collier, le volume occupé par son corps dans l'enceinte pléthysmographique ne varie alors qu'en fonction du volume d'air inspiré et expiré, de sorte que la mesure effectuée est caractéristique de la respiration de l'animal, le bruit du signal étant limité à un niveau très bas.

Le dessin annexé illustre, schématiquement et à titre d'exemple, une forme d'exécution de l'enceinte de mesure pléthysmographique objet de la présente invention.

La figure 1 est une vue latérale en coupe du corps tubulaire dans lequel est ménagée cette enceinte.

La figure 2 est une vue en coupe selon la ligne II-II de la figure 1.

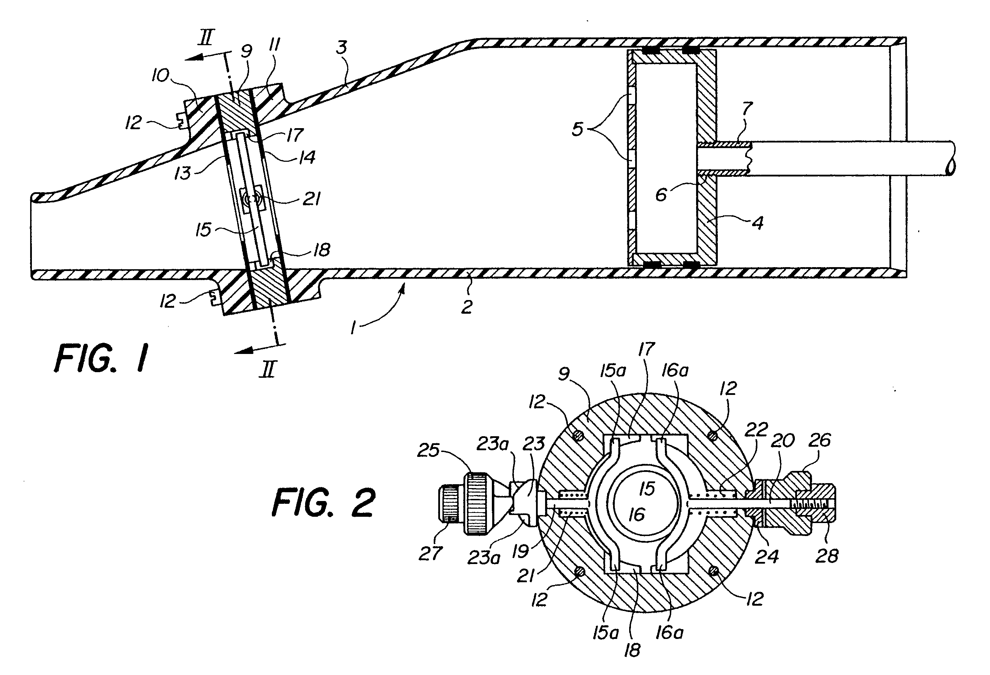

L'enceinte pléthysmographique 1 illustrée par la figure 1 comporte une partie cylindrique 2 et une partie avant en forme de tronc de cône rectangle 3. Le partie cylindrique 2 renferme un piston creux 4 qui comporte sur sa face interne des ouvertures 5 pour communiquer avec l'intérieur du corps tubulaire 1 et sur sa face extérieure une ouverture filetée 6 dans laquelle l'extrêmité d'un conduit 7 est vissée.

La partie tronconique 3 est divisée en deux par un élément annulaire 8 comprenant une bague 9 prise entre deux saillies annulaires 10 et 11 solidaires respectivement de deux tronçons de la partie tronconique 3 assemblées par des vis de serrage 12. Des feuilles d'un élastomère découpées en forme d'anneaux 13 et 14 sont serrées entre les deux faces respectives de la bague 8 et les saillies annulaires 9 et 10. Ces anneaux d'élastomère s'étendent radialement à l'intérieur de l'espace délimité par la partie tronconique 3. Les ouvertures de ces anneaux élastomère 13 et 14 sont destinées à laisser passer le museau du rongeur, ici un rat ou un cochon d'Inde en se déformant vers l'avant et en créant ainsi une barrière étanche. Le volume délimité par le piston 4 d'une part, et les anneaux 13 et 14 d'autre part, constitue l'enceinte pléthysmographique.

Dans l'espace séparant les anneaux d'élastomère 13 et 14 et délimité par la bague médiane 9, prend place un collier de retenue destiné à s'ajuster derrière la tête de l'animal. Ce collier comporte (fig 2) deux segments annulaires 15 et 16 qui se terminent par deux segments rectilignes 15a respectivement 16a dirigés vers l'extérieur. Les deux segments rectilignes d'un même segment annulaire sont situés sur une droite commune et s'étendent dans deux dégagements respectifs 17 et 18 diamétralement opposés ménagés dans la bague médiane 9 et s'étendant dans le plan de l'ouverture formée par ledit collier. Chacun des segments annulaires 15 et 16 est fixé dans sa partie médiane à deux tiges 19 respectivement 20 qui s'étendent radialement vers l'extérieur et montées coulissantes à travers la bague médiane 9. Deux ressorts en hélice 21, 22 sont enroulés autour des tiges 19, respectivement 20 et sont comprimés entre les segments annulaires respectifs 15 et 16 et la bague médiane 9, tendant ainsi toujours à rapprocher ces segments annulaires 15 et 16 l'un de l'autre.

Les tiges 19, 20 traversent à la sortie de la bague 9 deux plots cylindriques 23, 24 fixés en deux points diamètralement opposés de la bague 9 et présentant chacun deux cames 23a en forme de portions d'hélice, les cames du plot 24 ne sont pas visibles celui-ci étant dessiné en coupe. Les deux cames de chaque plot cylindrique sont diamètralement opposées par rapport à l'axe du plot. Deux organes d'actionnement molletés 25, 26 sont montés rotativement sur les tiges respectives 19, 20. Les parties des organes d'actionnement 25, 26 faisant face aux cames des plots respectifs 23, 24 présentent des cames hélicoïdales 25a complémentaires aux cames 23a, les cames de l'organe 26 n'étant pas visibles celui-ci étant dessiné en coupe.

Les tiges 19, 20 se terminent par des parties filetées qui se vissent dans des organes de butée 27, respectivement 28, qui, sous l'action des forces exercées par les ressorts 21 et 22 appliquent élastiquement les organes d'actionnement 25, 26 contre les plots respectifs 23, 24. Ce mécanisme permet donc, en faisant tourner les organes d'actionnement 25, 26 dans le sens approprié autour des tiges respectives 19, 20 de déplacer axialement ces tiges 19, 20 par le glissement des cames hélicoïdales 25a, 26a de ces organes d'actionnement contre les cames héliocoïdales fixes des plots 23, 24. Par conséquent, ces tiges 19, 20 peuvent chacune occuper deux positions radiales par rapport à la bague 9, commandant ainsi également deux positions des segments annulaires 15 et 16 correspondant à deux écartements de ces segments annulaires. Si la différence entre ces deux écartements est fixe et donnée par la longeur axiale des cames 23a, 24a, les organes de butée 27 et 28 permettent d'effectuer un réglage fin des positions respectives des segments annulaires 15 et 16 en vissant ou en dévissant les extrêmités des tiges 19 et 20 dans les organes de butée 27, respectivement 28. Ceci permet d'adapter l'ouverture minimum des segments annulaires en fonction de la taille de l'animal. L'ouverture du collier doit être inférieure au diamètre de sa tête sans que son cou ne soit comprimé.

Les segments rectilignes 15a, 16a prolongeant les segments annulaires 15, respectivement 16, engagés dans les dégagements 17 respectivement 18 de la bague médiane 9, sont de ce fait empêchés de tourner autour des axes des tiges respectives 19, 20 lors de la manoeuvre des organes de butée 27, respectivement 28.

La mise en place de l'animal dans l'enceinte pléthysmographique s'effectue en retirant le piston creux 4 de la partie cylindrique 2 et en écartant les segments annulaires 15 et 16 l'un de l'autre afin de ménager une ouverture maximum du collier. On introduit alors l'animal en engageant son museau à travers les ouvertures des anneaux d'élastomères 13 et 14, on réintroduit le piston creux 4 pour limiter le volume de l'enceinte au minimum nécessaire au corps de l'animal. On resserre alors l'ouverture du collier en manoeuvrant les organes d'actionnement 25 respectivement 26. A partir de ce moment, l'animal ne peut plus retirer sa tête dans l'enceinte s'étendant entre le piston 4 et les anneaux d'élastomère 13 et 14.

Le conduit 7 est alors relié à un transducteur de mesure (non représenté) et les tests respiratoires peuvent commencer.

## Revendications

1. Enceinte de mesure pléthysmographique destinée à recevoir le corps d'un animal de laboratoire en vue de mesurer différents paramètres respiratoires, cette enceinte comprenant un corps tubulaire (2) dont l'extrémité avant présente une section tronconique (3) se terminant par une ouverture d'admission d'un flux gazeux à inhaler, cette enceinte étant délimitée d'une part vers cette ouverture d'admission par deux joints annulaires (13,14) séparés axialement dont les ouvertures respectives sont destinées au passage du museau de l'animal, d'autre part par un piston (4) d'adaptation du volume de l'enceinte à la taille de l'animal, caractérisée par le fait qu'un collier de retenue est ménagé dans l'espace annulaire séparant les deux joints annulaires, ce collier de retenue comportant deux segments annulaires (15,16) fixés chacun par leur partie médiane à une tige (19,20) s'étendant radialement par rapport audit corps tubulaire et montée coulissante à travers la paroi de ce corps, un ressort de rappel en hélice (21,22) entourant la portion de chacune de ces tiges s'étendant entre les dites parties médianes et la paroi dudit corps tubulaire, les extrémités externes de ces tiges étant fixées à des organes de butée respectifs (27,28), une came étant montée rotativement autour de chacune de ces tiges entre l'organe de butée et une contre-came solidaire (23a) dudit corps tubulaires, ces cames étant susceptibles de définir deux positions axiales des tiges respectives à l'encontre de la pression desdits ressorts en hélice.

2. Enceinte de mesure pléthysmographique selon la revendication 1, caractérisée par le fait que lesdits segments annulaires composant ledit collier se terminent par deux segments rectilignes (15a,16a) situés sur une même droite et s'étendant vers l'extérieur, ces segments rectilignes étant engagés dans deux dégagements (17,18) de la paroi dudit corps tubulaire situés dans le plan de l'ouverture formée entre lesdits segments annulaires.

3. Enceinte de mesure pléthysmographique selon la revendication 1, caractérisée par le fait que lesdits organes de butée (27,28) respectifs sont vissés aux extrémités externes desdites tiges.

## Claims

1. A plethysmographic measuring chamber for receiving the body of a laboratory animal for measuring various respiratory parameters, the chamber comprising a tubular body (2) having a frusto-conical portion (3) at its front end terminating in an aperture for admitting a flow of gas for inhaling, the chamber being bounded at the inlet opening by two axially separated annular seals (13,14) having respective openings for receiving the animal's snout, whereas the other end of the chamber is bounded by a piston (4) for adjusting the volume of the chamber to the size of the animal, characterised in that a retaining collar is formed in the annular space between the two annular seals, the retaining collar comprising two annular segments (15,16) each secured by their central part to a rod (19,20) extending radially with respect to the tubular body and mounted for sliding across the wall of said body, a helical return spring (21,22) surrounding the portion of each rod extending between said central parts and the wall of the tubular body, the external ends of the rods being secured to respective abutment means (27,28) a cam being mounted for rotation around each rod between the abutment means and a cooperating cam secured (23a) to the tubular body, the cams being adapted to define two axial positions of the respective rods against the pressure of the helical springs.

2. A plethysmographic measuring chamber according to claim 1, characterised in that the annular segments making up the collar terminate in two straight segments (15a,16a) disposed on the same straight line and extending towards the exterior, the straight segments engaging in two recesses (17,18) in the tubular body wall and in the plane of the opening formed between the annular segments.

3. A plethysmographic measuring chamber according to claim 1, characterised in that the respective abutment means (27,28) are screwed to the outer ends of the rods.

## Patentansprüche

1. Volumenmeßkammer zur Aufnahme des Körpers eines Labortiers zur Messung verschiedener Atmungsparameter, die einen rohrförmigen Körper (2) besitzt, dessen vorderes Ende einen kegelstumpfförmigen Abschnitt (3) besitzt, an dessen Ende eine Eintrittsöffnung für einen zu inhalierenden Gasstrom vorgesehen ist, und die einerseits auf diese Eintrittsöffnung zu durch zwei ringförmige Dichtungen (13, 14), die axial voneinander getrennt sind und deren Öffnung jeweils für den Durchgang der Schnauze des Tiers bestimmt ist, und andererseits durch einen Kolben (4) zur Anpassung des Volumens der Kammer an die Größe des Tiers abgegrenzt ist, dadurch gekennzeichnet, daß in dem die beiden ringförmigen Dichtungen voneinander trennenden ringförmigen Raum ein Haltering vorgesehen ist, der zwei ringförmige Segmente (15, 16) besitzt, die jeweils mit ihrem mittleren Teil an einer Stange (19, 20) befestigt sind, die sich bezüglich des rohrförmigen Körpers radial erstreckt und in der Wand dieses Körpers verschiebbar montiert ist, daß eine Rückholschraubenfeder (21, 22) den Abschnitt jeder Stange umgibt, der sich zwischen diesen mittleren Teilen und der Wand des rohrförmigen Körpers erstreckt, daß die äußeren Enden der Stangen an Anschlagsorganen (27, 28) befestigt sind, daß ein Nokkenorgan drehbar um jede der Stangen zwischen dem Anschlagsorgan und einem an dem rohrförmigen Körper befestigten Gegennokkenorgan (23a) montiert ist und daß diese Nokkenorgane zwei axiale Stellungen der jeweiligen Stange entgegen dem Druck der Schraubenfedern definieren können.

2. Volumenmeßkammer nach Anspruch 1, dadurch gekennzeichnet, daß die den Haltering bildenden ringförmigen Segmente mit zwei geradlinigen Segmenten (15a, 16a) enden, die auf einer gemeinsamen Geraden liegen und sich nach außen erstrecken und in zwei Aussparungen (17, 18) in der Wand des rohrförmigen Körpers eintreten, die in der Ebene der zwischen den ringförmigen Segmenten gebildeten Öffnung angeordnet sind.

3. Volumenmeßkammer nach Anspruch 1, dadurch gekennzeichnet, daß die Anschlagsorgane (27, 28) auf die Außenenden der Stangen aufgeschraubt sind.

FIG. 1

FIG. 2